# EUROPEAN PATENT SPECIFICATION

(11) **EP 4 255 444 B1**
(45) Date of publication and mention of the grant of the patent: **15.07.2026**
(21) Application number: 21900200.3
(22) Date of filing: 03.12.2021
(51) Int. Cl.: A61K 31/702, A23L 33/125, A61P 25/00

(54) **HUMAN MILK OLIGOSACCHARIDES FOR USE IN SUPPORTING MATURATION OR IMPROVEMENT OF SLEEPING PATTERNS**
MUTTERMILCHOLIGOSACCHARIDE ZUR VERWENDUNG BEI DER UNTERSTÜTZUNG DER REIFUNG ODER VERBESSERUNG VON SCHLAFMUSTERN
OLIGOSACCHARIDES DE LAIT HUMAIN DESTINÉS À ÊTRE UTILISÉS POUR PERMETTRE LA MATURATION OU L'AMÉLIORATION DE RYTHMES DE SOMMEIL

(30) Priority: 04.12.2020 DK PA202001371
(43) Date of publication of application: 11.10.2023
(73) Proprietor: Glycom A/S, 2970 Hørsholm (DK)
(72) Inventor: ADMUNSEN, Ingvild Dybdrodt, 2450 Copenhagen SV (DK); GARCIA-RODENAS, Clara Lucia, 1072 Forel (CH); HAUSER, Jonas, 1003 Lausanne (CH)
(74) Representative: dsm-firmenich IP
(86) International application number: PCT/IB2021/061287
(87) International publication number: WO 2022/118269

(56) References cited:
- WO-A1-2016/066175
- WO-A1-2017/071715
- WO-A1-2017/129650
- WO-A1-2017/198276
- WO-A1-2019/087140
- WO-A1-2019/087140
- WO-A1-2019/123316
- WO-A1-2020/178774
- US-A1- 2020 330 492
- US-A1- 2020 330 493

## Description

### FIELD OF THE INVENTION

This invention relates to the non-therapeutic use of human milk oligosaccharides (HMOs) in a non-infant subject to support maturation or improvement of sleeping patterns wherein the maturation or improvement of sleeping patterns involves development of night and day sleep pattern from a more fragmented sleep composed of frequent short bout of sleeps and absence of circadian cycle to a pattern with long uninterrupted night sleep, limited day sleep and presence of circadian cycle. In particular, the HMO is a fucosylated HMO.

### BACKGROUND OF THE INVENTION

Preliminary evidence based on animal studies have implicated that human milk oligosaccharides (HMOs) could play a significant role in the development of the central nervous system and therefore in the associated functions.

Reduced sleep has been associated with multiple negative effects, such as decreased cognitive development, mood regulation, and overall health (L.J. Meltzer, J.A. Mindell Psychiatr. Clin. North Am. 29, 1059 (2006)). Specifically, short sleep duration and poor sleep quality has been associated with obesity (E.M. Taveras et al. Arch. Pediatr. Adolesc. Med. 162, 305 (2008)) and behavioural problems (B. Zuckerman et al. Pediatrics 80, 664 (1987)). The most common sleep disturbances in infants and children are those related to wakefulness (i.e. either difficulties in settling at bedtime or failure to sleep through the night without interruptions). It has been estimated that these disturbances affect 15 to 35 % of infants aged less than 24 months (France et al. Sleep Medicine Reviews 3, 265 (1999)). Furthermore, these sleep disturbances might look different at different age as sleep pattern mature in early life, switching from a more fragmented non-circadian sleep to long unbroken sleep session following a circadian rhythm (T.F. Anders et al. A longitudinal study of night-time sleep-wake patterns in infants from birth to one year In: J.D. Call et al. (eds): Frontiers of Infant Psychiatry. New York: Basic Books, pp. 50-166 (1983)).

WO2019/087140 relates to a human milk oligosaccharide (HMO) for use in reducing or preventing prolonged fatigue which may be caused by a disease or condition such as sleep deprivation or sleep disorders.

US2020/330492 relates to a method of treating serotonin and/or tryptophan dysregulation by administering one or more human milk oligosaccharides which may be used to treat a list long of symptoms, diseases or conditions including insomnia.

US2020/330493 discloses compositions comprising 6'silalylactose (6'SL) and lacto-N-tetraose (LNT), for use in the reduction of nociception in an infant or young child, which may contribute to reducing the crying periods and to improving the quality of sleep. The potential impact of HMOs on gut microbiota have been implicated since HMOs are largely metabolized in the intestinal tracts. In contrast, the potential effects of HMOs on brain functional development are less studied, and most studies in this field focused on learning and memory. For example, using chronic oral administration of 2'-FL rodents, Vazquez et al. J. Nutr. Biochem. 26, 455 (2015) demonstrated that the treated animals exhibit significantly better learning and working memory functions. Nevertheless, to the best of our knowledge, the present study is the first study reporting human results and investigating impact of HMO on maturation of sleep patterns or trouble sleeping.

There is a need to deliver such health benefits in the non-infant in a manner that does not induce side effects and/or in a manner that is easy to deliver, and well accepted by the patients or health care practitioners. There is also a need to deliver such benefits in a manner that does keep the cost of such delivery reasonable and affordable by most.

### SUMMARY OF THE INVENTION

In a first aspect, this invention provides for non-therapeutic use of one or more fucosylated human milk oligosaccharides (HMOs) to support maturation or improvement of sleeping patterns, in a non-infant human, wherein the maturation or improvement of sleeping patterns involves development of night and day sleep pattern from a more fragmented sleep composed of frequent short bout of sleeps and absence of circadian cycle to a pattern with long uninterrupted night sleep, limited day sleep and presence of circadian cycle.

In one embodiment, the fucosylated human milk oligosaccharides (HMOs) or a nutritional composition comprising the fucosylated human milk oligosaccharides (HMOs) are used in the non-therapeutic dietary management of a non-infant human having fragmented sleep composed of frequent short bout of sleeps and absence of circadian cycle.

Preferably, the one or more fucosylated HMOs are fucosylated neutral HMOs.

Preferably, the non-infant human is administered the one or more fucosylated HMOs for a period of at least 1 week, more preferably for at least 2 weeks. For example, the human can be administered the one or more fucosylated HMOs for a period of at least 4 weeks or even more.

The non-infant human may be administered a dose of one or more fucosylated HMOs of about 0.1 to 10 g per day. In one embodiment, the non-infant human may be administered a higher dose initially followed by a lower dose of one or more fucosylated HMOs. The higher dose is preferably about 0.5 g to about 10 g per day (for example about 1 g to about 7.5 g per day) and the lower dose is preferably about 0.1 g to about 5 g per day (for example about 0.2 g to about 3 g per day).

In any of the aspects mentioned above, the non-infant suffering from trouble sleeping is for example an IBS-patient.

### DETAILED DESCRIPTION OF THE INVENTION

As used herein, the following terms have the following meanings.

"Dietary management" means exclusive or partial feeding of patients who, because of a disease, disorder or medical condition are suffering from:
- either have a limited, impaired or disturbed capacity to take, digest, absorb, metabolise or excrete ordinary food or certain nutrients contained therein, or metabolites, or
- have other medically-determined nutrient requirements
(see: Commission Notice on the classification of Food for Special Medical Purposes of the European Commission, Official Journal of the European Union C 401, 25.11.2017, p. 10-11).

"Enteral administration" means any conventional form for delivery of a composition to a human that causes the deposition of the composition in the gastrointestinal tract (including the stomach). Methods of enteral administration include feeding through a naso-gastric tube or jejunum tube, oral, sublingual and rectal.

"Effective amount" means an amount of a composition that provides a HMO in a sufficient amount to render a desired treatment outcome in a human. An effective amount can be administered in one or more doses to achieve the desired treatment outcome.

"FODMAP" means fermentable oligosaccharides, disaccharides, monosaccharides and polyols.

"Human milk oligosaccharide" or "HMO" means a complex carbohydrate found in human breast milk (Urashima et al.: Milk Oligosaccharides. Nova Science Publisher (2011); Chen Adv. Carbohydr. Chem. Biochem. 72, 113 (2015)). The HMOs have a core structure comprising a lactose unit at the reducing end that can be elongated by one or more β-N-acetyl-lactosaminyl and/or one or β-more lacto-N-biosyl units, and which core structure can be substituted by an α L-fucopyranosyl and/or an α-N-acetyl-neuraminyl (sialyl) moiety. In this regard, the non-acidic (or neutral) HMOs are devoid of a sialyl residue, and the acidic HMOs have at least one sialyl residue in their structure. The non-acidic (or neutral) HMOs can be fucosylated or non-fucosylated. Examples of such neutral non-fucosylated HMOs include lacto-N-tetraose (LNT), lacto-N-neotetraose (LNnT), lacto-N-neohexaose (LNnH), para-lacto-N-neohexaose (pLNnH), para-lacto-N-hexaose (pLNH) and lacto-N-hexaose (LNH). Examples of neutral fucosylated HMOs include 2'-fucosyllactose (2'-FL), lacto-N-fucopentaose I (LNFP-I), lacto-N-difucohexaose I (LNDFH-I), 3-fucosyllactose (3-FL), difucosyllactose (DFL), lacto-N-fucopentaose II (LNFP-II), lacto-N-fucopentaose III (LNFP-III), lacto-N-difucohexaose III (LNDFH-III), fucosyl-lacto-N-hexaose II (FLNH-II), lacto-N-fucopentaose V (LNFP-V), lacto-N-fucopentaose VI (LNFP-VI), lacto-N-difucohexaose II (LNDFH-II), fucosyl-lacto-N-hexaose I (FLNH-I), fucosyl-para-lacto-N-hexaose I (FpLNH-I), fucosyl-para-lacto-N-neohexaose II (FpLNnH II) and fucosyl-lacto-N-neohexaose (FLNnH). Examples of acidic HMOs include 3'-sialyllactose (3'-SL), 6'-sialyllactose (6'-SL), 3-fucosyl-3'-sialyllactose (FSL), LST a, fucosyl-LST a (FLST a), LST b, fucosyl-LST b (FLST b), LST c, fucosyl-LST c (FLST c), sialyl-LNH (SLNH), sialyl-lacto-N-hexaose (SLNH), sialyl-lacto-N-neohexaose I (SLNH-I), sialyl-lacto-N-neohexaose II (SLNH-II) and disialyl-lacto-N-tetraose (DSLNT).

"Irritable bowel syndrome" (abbreviated as IBS) means a functional bowel disorder in which recurrent abdominal pain is associated with defecation or a change in bowel habits. Disordered bowel habits are typically present (i.e., constipation, diarrhoea or a mix of constipation and diarrhoea), as are symptoms of abdominal bloating/distension. When diagnosed under Rome IV criteria, the patient must experience recurrent abdominal pain on average at least 1 day/week in the last 3 months, and the pain must be associated with two or more of the following criteria: (1) related to defecation; (2) associated with a change in the frequency of stool; and (3) associated with a change in the form (appearance) of stool. Symptom onset should occur at least 6 months prior to diagnosis and symptoms should be present during the last 3 months.

"Microbiota", "microflora" and "microbiome" mean a community of living microorganisms that typically inhabits a bodily organ or part, particularly the gastro-intestinal organs of humans. The most dominant members of the gastrointestinal microbiota include microorganisms of the phyla of Firmicutes, Bacteroidetes, Actinobacteria, Proteobacteria, Synergistetes, Verrucomicrobia, Fusobacteria, and Euryarchaeota, at genus level Bacteroides, Faecalibacterium, Bifidobacterium, Roseburia, Alistipes, Collinsella, Blautia, Coprococcus, Ruminococcus, Eubacterium and Dorea, at species level Bacteroides uniformis, Alistipes putredinis, Parabacteroides merdae, Ruminococcus bromii, Dorea longicatena, Bacteroides caccae, Bacteroides thetaiotaomicron, Eubacterium hallii, Ruminococcus torques, Faecalibacterium prausnitzii, Ruminococcus lactaris, Collinsella aerofaciens, Dorea formicigenerans, Bacteroides vulgatus and Roseburia intestinalis. The gastrointestinal microbiota includes the mucosa-associated microbiota, which is located in or attached to the mucous layer covering the epithelium of the gastrointestinal tract, and luminal-associated microbiota, which is found in the lumen of the gastrointestinal tract.

"Modulating of microbiota" means exerting a modifying or controlling influence on microbiota, for example an influence leading to an increase in the indigenous intestinal abundance of Bifidobacterium, Barnesiella, Faecalibacterium and/or butyrate producing bacteria. In another example, the influence may lead to a reduction of the intestinal abundance of Ruminococcus gnavus and/or Proteobacteria. "Proteobacteria" are a phylum of Gram-negative bacteria and include a wide variety of pathogenic bacteria, such as Escherichia, Salmonella, Vibrio, Helicobacter, Yersinia and many other notable genera.

"Non-infant human" or "non-infant" means a human of 3 years of age and older. A non-infant human can be a child, a teenager, an adult or an elderly person, preferably a teenager, an adult or an elderly person, more preferably an adult or an elderly person.

"Oral administration" means any conventional form for the delivery of a composition to a human through the mouth. Accordingly, oral administration is a form of enteral administration.

"Sleep maturation or maturation of sleeping patterns" refers to improving trouble sleeping, for example the development of night and day sleep pattern from a more fragmented sleep composed of frequent short bout of sleeps and absence of circadian cycle to a pattern with long uninterrupted night sleep, limited day sleep and presence of circadian cycle.

"Synthetic composition" means a composition which is artificially prepared and preferably means a composition containing at least one compound that is produced ex vivo chemically and/or biologically, e.g. by means of chemical reaction, enzymatic reaction or recombinantly The synthetic composition typically comprises one or more HMOs. Also, in some embodiments, the synthetic compositions may comprise one or more nutritionally or pharmaceutically active components which do not affect adversely the efficacy of the HMOs. Some non-limiting embodiments of a synthetic composition of the invention are described below.

The fucosylated HMOs used in the synthetic composition, pack and method of the invention can be isolated or enriched by well-known processes from milk(s) secreted by mammals including, but not limited to human, bovine, ovine, porcine, or caprine species. HMOs can also be produced by well-known processes using microbial fermentation, enzymatic processes, chemical synthesis, or combinations of these technologies. As examples, 2'-FL can be made chemically as described in WO 2010/115934 and WO 2010/115935. As examples of enzymatic production, sialylated oligosaccharides can be made as described in WO 2012/007588, fucosylated oligosaccharides can be made as described in WO 2012/127410, and advantageously diversified blends of human milk oligosaccharides can be made as described in WO 2012/156897 and WO 2012/156898. Biotechnological methods which describe how to make core (non-fucosylated neutral) human milk oligosaccharides optionally substituted by fucose or sialic acid using genetically modified E. coli con be found in WO 01/04341 and WO 2007/101862. In particular, WO2015/032413, WO2015/032412 and WO2016/095924 describes biotechnological processes for manufacture of 2'-FL and DFL.

The fucosylated neutral HMO may be complemented with other human milk oligosaccharides. For example, the fucosylated neutral HMO may be complemented with a non-fucosylated HMO, for example LNT and/or LNnT.

The fucosylated neutral HMO, optionally in combination with a non-fucosylated HMO, can be used as it is or they are (neat), without any carrier and/or diluent. In other embodiment, the fucosylated neutral HMO(s), optionally in combination with a non-fucosylated HMO, is/are used in a synthetic composition with one or more inert carriers/diluents that are acceptable in nutritional or pharmaceutical compositions, for example solvents (e.g. water, water/ethanol, oil, water/oil), dispersants, coatings, absorption promoting agents, controlled release agents, inert excipients (e.g. starches, polyols, granulating agents, microcrystalline cellulose, diluents, lubricants, binders, disintegrating agents). These compositions do not contain prebiotic and/or probiotic [that is: a synthetic composition consisting of a) a fucosylated neutral HMO, optionally in combination with a non-fucosylated HMO, b) one or more inert carriers/diluents that are acceptable in nutritional or pharmaceutical compositions]. In other embodiment, the fucosylated neutral HMO(s), optionally in combination with a non-fucosylated HMO, is/are used in a synthetic pharmaceutical or nutritional composition that may contain a prebiotic and/or probiotic.

In this regard, the following embodiments for use in improving sleep maturation, for example improving trouble sleeping, in a non-infant are especially comprised:
- a single HMO which is selected from the group consisting of 2'-FL, 3-FL, DFL, LNFP-I, LNFP-II, LNFP-III, LNFP-V and LNFP-VI,
- exactly two HMOs selected from the group consisting of 2'-FL, 3-FL, DFL, LNFP-I, LNFP-II, LNFP-III, LNFP-V and LNFP-VI,
- a fucosylated neutral HMO which is selected from the group consisting of 2'-FL, 3-FL, DFL, LNFP-I, LNFP-II, LNFP-III, LNFP-V and LNFP-VI and at least a further non-fucosylated neutral HMO,
- a synthetic composition comprising, consisting of or consisting essentially of a fucosylated neutral HMO selected from the group consisting of 2'-FL, 3-FL, DFL, LNFP-I, LNFP-II, LNFP-III, LNFP-V, LNFP-VI and combination thereof.

In one preferred embodiment, the HMO is 2'-FL. In other preferred embodiment, the HMO is DFL. In other preferred embodiment, the HMO is 3-FL. In other preferred embodiment, the HMO is LNFP-I. In other preferred embodiment, the HMO is a combination of 2'-FL and DFL. In other preferred embodiment, the HMO is a combination of 2'-FL and LNFP-I. In other preferred embodiment, the HMO is a combination of 2'-FL and (LNT and/or LNnT) [that is: 2'-FL and LNT; 2'-FL and LNnT; 2'-FL, LNT and LNnT]. In other preferred embodiment, the HMO is a combination of 2'-FL, DFL and (LNT and/or LNnT) [that is: 2'-FL, DFL and LNT; 2'-FL, DFL and LNnT; 2'-FL, DFL, LNT and LNnT]. More preferably, the HMO is 2'-FL, optionally in combination with LNnT.

Concerning the above preferred and more preferred embodiments, the preferred non-infant humans are selected from the group consisting of a child, a teenager, an adult and an elderly person, more preferably selected from the group consisting of a teenager, an adult and an elderly person, even more preferably selected from the group consisting of an adult and an elderly person. In one embodiment of the non-infant humans, the child, teenager, adult or elderly person, more preferably the teenager, an adult or an elderly person, even more preferably the adult or elderly person is an irritable bowel syndrom (IBS) patient.

Furthermore, the preferred embodiments of the daily doses comprised in the pack according to the third aspect are those disclosed above.

Furthermore, the preferred embodiments of the use in the dietary management of a non-infant human according to the fourth aspect are those disclosed above.

Furthermore, the preferred embodiments of the method according to the fifth aspect are those disclosed above.

The synthetic composition can be in the form of a nutritional composition. For example, the nutritional composition can be a food composition, a rehydration solution, a medical food or food for special medical purposes, a nutritional supplement and the like. The nutritional composition can contain sources of protein, lipids and/or digestible carbohydrates and can be in powdered or liquid forms. The composition can be designed to be the sole source of nutrition or as a nutritional supplement.

Suitable protein sources include milk proteins, soy protein, rice protein, pea protein and oat protein, or mixtures thereof. Milk proteins can be in the form of milk protein concentrates, milk protein isolates, whey protein or casein, or mixtures of both. The protein can be whole protein or hydrolysed protein, either partially hydrolysed or extensively hydrolysed. Hydrolysed protein offers the advantage of easier digestion which can be important for humans with inflamed or compromised GI tracts. The protein can also be provided in the form of free amino acids. The protein can comprise about 5 % to about 30 % of the energy of the nutritional composition, normally about 10 % to 20 %.

The protein source can be a source of glutamine, threonine, cysteine, serine, proline, or a combination of these amino acids. The glutamine source can be a glutamine dipeptide and/or a glutamine enriched protein. Glutamine can be included due to the use of glutamine by enterocytes as an energy source. Threonine, serine and proline are important amino acids for the production of mucin. Mucin coats the gastrointestinal tract and can improve intestinal barrier function and mucosal healing. Cysteine is a major precursor of glutathione, which is key for the antioxidant defences of the body.

Suitable digestible carbohydrates include maltodextrin, hydrolysed or modified starch or corn starch, glucose polymers, corn syrup, corn syrup solids, high fructose corn syrup, rice-derived carbohydrates, pea-derived carbohydrates, potato-derived carbohydrates, tapioca, sucrose, glucose, fructose, sucrose, lactose, honey, sugar alcohols (e.g. maltitol, erythritol, sorbitol), or mixtures thereof. In one embodiment, the composition is reduced in or free from added lactose or other FODMAP carbohydrates. Generally digestible carbohydrates provide about 35 % to about 55 % of the energy of the nutritional composition. A particularly suitable digestible carbohydrate is a low dextrose equivalent (DE) maltodextrin.

Suitable lipids include medium chain triglycerides (MCT) and long chain triglycerides (LCT). Preferably the lipid is a mixture of MCTs and LCTs. For example, MCTs can comprise about 30 % to about 70 % by weight of the lipids, more specifically about 50 % to about 60 % by weight. MCTs offer the advantage of easier digestion which can be important for humans with inflamed or compromised GI tracts. Generally, the lipids provide about 35 % to about 50 % of the energy of the nutritional composition. The lipids can contain essential fatty acids (omega-3 and omega-6 fatty acids). Preferably these polyunsaturated fatty acids provide less than about 30 % of total energy of the lipid source.

Suitable sources of long chain triglycerides are rapeseed oil, sunflower seed oil, palm oil, soy oil, milk fat, corn oil, high oleic oils, and soy lecithin. Fractionated coconut oils are a suitable source of medium chain triglycerides. The lipid profile of the nutritional composition is preferably designed to have a polyunsaturated fatty acid omega-6 (n-6) to omega-3 (n-3) ratio of about 4:1 to about 10:1. For example, the n-6 to n-3 fatty acid ratio can be about 6:1 to about 9:1.

The nutritional composition may also include vitamins and minerals. If the nutritional composition is intended to be a sole source of nutrition, it preferably includes a complete vitamin and mineral profile. Examples of vitamins include vitamins A, B-complex (such as B1, B2, B6 and B12), C, D, E and K, niacin and acid vitamins such as pantothenic acid, folic acid and biotin. Examples of minerals include calcium, iron, zinc, magnesium, iodine, copper, phosphorus, manganese, potassium, chromium, molybdenum, selenium, nickel, tin, silicon, vanadium and boron.

The nutritional composition can also include a carotenoid such as lutein, lycopene, zeaxanthin, and beta-carotene. The total amount of carotenoid included can vary from about 0.001 µg/ml to about 10 µg/ml. Lutein can be included in an amount of from about 0.001 µg/ml to about 10 µg/ml, preferably from about 0.044 µg/ml to about 5 µg/ml of lutein. Lycopene can be included in an amount from about 0.001 µg/ml to about 10 µg/ml, preferably about 0.0185 µg/ml to about 5 µg/ml of lycopene. Beta-carotene can comprise from about 0.001 µg/ml to about 10 mg/ml, for example about 0.034 µg/ml to about 5 µg/ml of beta-carotene.

The nutritional composition preferably also contains reduced concentrations of sodium, for example, from about 300 mg/l to about 400 mg/l. The remaining electrolytes can be present in concentrations set to meet needs without providing an undue renal solute burden on kidney function. For example, potassium is preferably present in a range of about 1180 to about 1300 mg/l, and chloride is preferably present in a range of about 680 to about 800 mg/l.

The nutritional composition can also contain various other conventional ingredients such as preservatives, emulsifying agents, thickening agents, buffers, fibres and prebiotics (e.g. fructooligosaccharides, galactooligosaccharides), probiotics (e.g. B. animalis subsp. lactis BB-12, B. lactis HN019, B. lactis Bi07, B. infantis ATCC 15697, L. rhamnosus GG, L. rhamnosus HNOOI, L. acidophilus LA-5, L. acidophilus NCFM, L. fermentum CECT5716, B. longum BB536, B. longum AH1205, B. longum AH1206, B. breve M-16V, L. reuteri ATCC 55730, L. reuteri ATCC PTA-6485, L. reuteri DSM 17938), antioxidant/anti-inflammatory compounds including tocopherols, carotenoids, ascorbate/vitamin C, ascorbyl palmitate, polyphenols, glutathione, and superoxide dismutase (melon), other bioactive factors (e.g. growth hormones, cytokines, TFG-β), colorants, flavours, and stabilisers, lubricants, and so forth.

The nutritional composition is, in one embodiment, free from gluten.

The nutritional composition can be formulated as a soluble powder, a liquid concentrate, or a ready-to-use formulation. The composition can be fed to a human in need via a nasogastric tube or orally. Various flavours, fibres and other additives can also be present.

The nutritional compositions can be prepared by any commonly used manufacturing techniques for preparing nutritional compositions in solid or liquid form. For example, the composition can be prepared by combining various feed solutions. A protein-in-fat feed solution can be prepared by heating and mixing the lipid source and then adding an emulsifier (e.g. lecithin), fat soluble vitamins, and at least a portion of the protein source while heating and stirring. A carbohydrate feed solution is then prepared by adding minerals, trace and ultra-trace minerals, thickening or suspending agents to water while heating and stirring. The resulting solution is held for 10 minutes with continued heat and agitation before adding carbohydrates (e.g. the HMOs and digestible carbohydrate sources). The resulting feed solutions are then blended together while heating and agitating and the pH adjusted to 6.6-7.0, after which the composition is subjected to high-temperature short-time processing during which the composition is heat treated, emulsified and homogenized, and then allowed to cool. Water soluble vitamins and ascorbic acid are added, the pH is adjusted to the desired range if necessary, flavours are added, and water is added to achieve the desired total solid level.

For a liquid product, the resulting solution can then be aseptically packed to form an aseptically packaged nutritional composition. In this form, the nutritional composition can be in ready-to-feed or concentrated liquid form. Alternatively, the composition can be spray-dried and processed and packaged as a reconstitutable powder.

When the nutritional product is a ready-to-feed nutritional liquid, it may be preferred that the total concentration of the sialylated and/or the non-fucosylated neutral HMO in the liquid, by weight of the liquid, is from about 0.1 % to about 1.5 %, including from about 0.2 % to about 1.0 %, for example from about 0.3 % to about 0.7 %. When the nutritional product is a concentrated nutritional liquid, it may be preferred that the total concentration of the sialylated and/or the non-fucosylated neutral HMO in the liquid, by weight of the liquid, is from about 0.2 % to about 3.0 %, including from about 0.4 % to about 2.0 %, for example from about 0.6 % to about 1.5 %.

In another embodiment, the nutritional composition is in a unit dosage form. The unit dosage form can contain an acceptable food-grade carrier, e.g. phosphate buffered saline solution, mixtures of ethanol in water, water and emulsions such as an oil/water or water/oil emulsion, as well as various wetting agents or excipients. The unit dosage form can also contain other materials that do not produce an adverse, allergic or otherwise unwanted reaction when administered to a human. The carriers and other materials can include solvents, dispersants, coatings, absorption promoting agents, controlled release agents, and one or more inert excipients, such as starches, granulating agents, microcrystalline cellulose, diluents, lubricants, binders, and disintegrating agents. Preferably carriers and other materials are low in FODMAPs or contain no FODMAPs. Preferably, the unit dosage form comprises primarily the sialylated and/or the non-fucosylated neutral HMO with a minimum amount of binders and/or excipients. Unit dosage forms are particularly suitable when nutritionally incomplete or not intended as a sole source of nutrition.

A unit dosage form of this invention can be administered orally, e.g. as a tablet, capsule, or pellet containing a predetermined amount of the mixture, or as a powder or granules containing a predetermined concentration of the mixture or a gel, paste, solution, suspension, emulsion, syrup, bolus, electuary, or slurry, in an aqueous or non-aqueous liquid, containing a predetermined concentration of the mixture. An orally administered composition can include one or more binders, lubricants, inert diluents, flavouring agents, and humectants. An orally administered composition such as a tablet can optionally be coated and can be formulated to provide sustained or controlled release of the sialylated and/or the non-fucosylated neutral HMO.

A unit dosage form of this invention can also be administered by naso-gastric tube or direct infusion into the GI tract or stomach.

A unit dosage form of this invention can also include therapeutic agents such as antibiotics, probiotics, analgesics, and anti-inflammatory agents.

The proper dosage of such a composition for a human can be determined in a conventional manner, based upon factors such as the human's condition, immune status, body weight and age. In some cases, the dosage will be at a concentration similar to that found for the fucosylated neutral HMOs of the composition in human breast milk. The required amount would generally be in the range from about 0.1 g to about 10 g per day, in certain embodiments from about 0.2 g to about 7.5 g per day, for example about 1 g to about 5 g per day. Appropriate dose regimes can be determined by methods known to those skilled in the art.

In further embodiment, the-fucosylated neutral HMO can be formulated as a pharmaceutical composition. The pharmaceutical composition can contain a pharmaceutically acceptable carrier, e.g. phosphate buffered saline solution, mixtures of ethanol in water, water and emulsions such as an oil/water or water/oil emulsion, as well as various wetting agents or excipients. The pharmaceutical composition can also contain other materials that do not produce an adverse, allergic or otherwise unwanted reaction when administered to humans. The carriers and other materials can include solvents, dispersants, coatings, absorption promoting agents, controlled release agents, and one or more inert excipients, such as starches, granulating agents, microcrystalline cellulose, diluents, lubricants, binders, and disintegrating agents. Preferably carriers and other materials are low in FODMAPs or contain no FODMAPs.

The pharmaceutical compositions can be administered orally, e.g. as a tablet, capsule, or pellet containing a predetermined amount, or as a powder or granules containing a predetermined concentration or a gel, paste, solution, suspension, emulsion, syrup, bolus, electuary, or slurry, in an aqueous or non-aqueous liquid, containing a predetermined concentration. Orally administered compositions can include binders, lubricants, inert diluents, flavouring agents, and humectants. Orally administered compositions such as tablets can optionally be coated and can be formulated to provide sustained, delayed or controlled release of the mixture therein.

The pharmaceutical compositions can also be administered by rectal suppository, aerosol tube, naso-gastric tube or direct infusion into the GI tract or stomach.

The pharmaceutical compositions can also include therapeutic agents such as antibiotics, probiotics, analgesics, and anti-inflammatory agents.

The proper dosage of these compositions for a human can be determined in a conventional manner, based upon factors such condition, immune status, body weight and age. In some cases, the dosage will be at a concentration similar to that found for the fucosylated neutral HMOs in human breast milk. The required amount would generally be in the range from about 0.1 g to about 10 g per day, in certain embodiments from about 0.2 g to about 7.5 g per day, for example from about 1 g to about 5 g per day. Appropriate dose regimes can be determined by conventional methods.

The amount of the fucosylated neutral HMO required to be administered will vary depending upon factors such as the risk and severity of the underlying condition, any other medical conditions or diseases, age, the form of the composition, and other medications being administered. Further the amount may vary depending upon whether the combination is being used to deliver a direct effect (when the dose may be higher) or whether the combination is being used as a secondary prevention/maintenance (when the dose may be lower). However, the required amount can be readily set by a medical practitioner and would generally be in the range from about 0.1 g to about 10 g per day, in certain embodiments from about 0.2 g to about 7.5 g per day, for example from about 1 g to about 5 g per day. An appropriate dose can be determined based on several factors, including, for example, body weight and/or condition, the severity of the underlying condition being treated or prevented, other ailments and/or diseases, the incidence and/or severity of side effects and the manner of administration. Appropriate dose ranges may be determined by methods known to those skilled in the art. During an initial treatment phase, the dosing can be higher (for example 0.5 g to 10 g per day, preferably 1 g to 7.5 g per day). During a maintenance phase, the dosing can be reduced (for example, 0.1 g to 5 g per day, preferably 0.2 g to 3 g per day).

### EXAMPLES

Examples are now described to further illustrate the invention:

### Example 1 - Nutritional composition

A ready to feed nutritional composition is prepared from water, maltodextrin, enzymatically hydrolysed whey protein (from cows milk), medium chain triglycerides (from coconut and/or palm kernel oil), corn-starch, soybean oil, soy lecithin, 2'-FL, magnesium chloride, calcium phosphate, guar gum, sodium ascorbate, potassium citrate, sodium phosphate, calcium citrate, choline chloride, potassium chloride, sodium citrate, magnesium oxide, taurine, L-carnitine, alpha-tocopheryl acetate, zinc sulphate, ferrous sulphate, niacinamide, calcium pantothenate, vitamin A palmitate, citric acid, manganese sulphate, pyridoxine hydrochloride, vitamin D3, copper sulphate, thiamine mononitrate, riboflavin, beta carotene, folic acid, biotin, potassium iodide, chromium chloride, sodium selenate, sodium molybdate, phytonadione, vitamin B12.

The composition has a calorific density of 1.0 kcal/ml with a caloric distribution (% of kcal) as follows: protein: 16 %, carbohydrate: 51 %, fat: 33 %. The protein source has an NPC:N ratio of 131:1. The MCT:LCT ratio is 70:30 and the n6:n3 ratio is 7.4:1. The osmolality (mOsm/kg water) is 270 when unflavoured. The composition contains 85 % water and 1500 ml meets 100 % of the RDI for 22 key micronutrients.

The composition has a balanced peptide profile which promotes GI absorption and integrity and the enzymatically hydrolysed 100 % whey protein facilitates gastric emptying. The MCT decreases the potential for fat malabsorption. The composition is nutritionally complete for tube feeding or oral supplementation.

### Example 2 - Tablet composition

A tablet is prepared from 2'-FL (or from 2'-FL:LNnT 4:1 by weight), hydroxypropyl methylcellulose, sodium alginate, gum, microcrystalline cellulose, colloidal silicon dioxide, and magnesium stearate. All raw materials except the magnesium stearate are placed into a high shear granulator and premixed. Water is sprayed onto the premix while continuing to mix at 300 rpm. The granulate is transferred to a fluidised bed drier and dried at 75 °C. The dried powder is sieved and sized using a mill. The resulting powder is then lubricated with magnesium stearate and pressed into tablets. The tablets each contain 325 mg of 2'-FL (or 2'-FL:LNnT 4:1 by weight). The tablets each have a weight of 750 mg.

### Example 3 - Capsule composition

A capsule is prepared by filling about 1 g of 2'-FL (or 2'-FL:LNnT 4:1 by weight) into a 000-gelatine capsule using a filing machine. The capsules are then closed. The 2'-FL (or 2'-FL:LNnT 4:1 by weight) is in free flowing, powder form.

### Example 4 - Nutritional composition

2'-FL (or 2'-FL:LNnT 4:1 by weight) is introduced into a rotary blender. An amount of 0.25 w% of silicon dioxide is introduced into the blender and the mixture blended for 10 minutes. The mixture is then agglomerated in a fluidised bed and filled into 5 gram stick packs and the packs are sealed.

### Example 5 - Human trial

In a single-armed, open-label trial, a total of 317 male and female patients were recruited to participate in the study who completed the baseline procedures. Patients were eligible to participate if: they were 18 years of age or older; met the Rome IV Irritable Bowel Syndrome (IBS) diagnostic criteria; ability to read, spoke and understood English; and were able and willing to understand and comply with the study procedures.

Patients were excluded if: they had participated in a clinical study one month prior to screening visit; they were diagnosed with celiac disease, Crohn's disease, ulcerative colitis, diverticulitis, inflammatory bowel disease, or *Clostridium difficile* infection by a medical doctor; they were pregnant or lactating or wished to become pregnant during the period of the study; or had a lack of suitability for participation in the study for any reason as judged by the site investigator or Principal Investigator.

At an initial screening visit, patients were given both written and oral information about the study and they were asked to sign the informed consent form. Patients were evaluated by a full review of clinical history and IBS diagnostic criteria were assessed. After inclusion in the trial, the patients filled out an electronic baseline survey.

The study intervention lasted for 12 weeks. Upon completion of the baseline survey, the investigational product for the first eight weeks of the study was sent to the patient by mail, whereas the investigational product for the remaining four weeks was sent after the patient completed the follow up survey after four weeks of intervention. The investigational product consisted of a daily dose of 5 grams of a combination of 2'-FL and LNnT in a 4:1 weight ratio, provided as white powder in single serve stick packs. The patients were asked to not change their normal diet during the study. During the intervention, the recruited patients were to complete three follow up online surveys every fourth week. The online surveys consisted of several verified questionnaires including PHQ-12 (12 item Patient Health Questionnaire; an instrument for quantifying the severity of non-gastrointestinal physical symptoms).

The question: "In the past 4 weeks, how much have you been bothered by trouble sleeping?" was rated on a scale from 0='not bothered at all' to 2='bothered a lot'. Patients reported a significant improvement in the symptom 'trouble sleeping' after 4 weeks of intervention, and after 12 weeks the symptom improved by 35% (see table below).

| | Baseline | Week 4 | Week 8 | Week 12 |
|---|---|---|---|---|
| mean | 1.04 | 0.60 | 0.65 | 0.67 |
| standard deviation | 0.72 | 0.68 | 0.71 | 0.66 |

It can be concluded that daily supplementation with 5 grams of a combination of 2'-FL and LNnT significantly improved sleep trouble in patients.

intervention, the recruited patients were to complete three follow up online surveys every fourth week. The online surveys consisted of several verified questionnaires including PHQ-12 (12 item Patient Health Questionnaire; an instrument for quantifying the severity of non-gastrointestinal physical symptoms).

The question: "In the past 4 weeks, how much have you been bothered by trouble sleeping?" was rated on a scale from 0='not bothered at all' to 2='bothered a lot'. Patients reported a significant improvement in the symptom 'trouble sleeping' after 4 weeks of intervention, and after 12 weeks the symptom improved by 35% (see table below).

| | Baseline | Week 4 | Week 8 | Week 12 |
|---|---|---|---|---|
| mean | 1.04 | 0.60 | 0.65 | 0.67 |
| standard deviation | 0.72 | 0.68 | 0.71 | 0.66 |

It can be concluded that daily supplementation with 5 grams of a combination of 2'-FL and LNnT significantly improved sleep trouble in patients.

## Claims

1. Non-therapeutic use of a fucosylated human milk oligosaccharide (HMO), preferably a fucosylated neutral HMO, to support maturation or improvement of sleeping patterns, in a non-infant human, wherein the maturation or improvement of sleeping patterns involves development of night and day sleep pattern from a more fragmented sleep composed of frequent short bout of sleeps and absence of circadian cycle to a pattern with long uninterrupted night sleep, limited day sleep and presence of circadian cycle.

2. The non-therapeutic use according to claim 1, wherein the fucosylated HMO is 2'-FL, 3-FL, DFL, LNFP-I, LNFP-II, LNFP-III, LNFP-V, LNFP-VI or any combination thereof.

3. The non-therapeutic use according to claim 1 or 2, wherein the fucosylated HMO is 2'-FL.

4. The non-therapeutic use according to claim 1 or 2, wherein the fucosylated HMO is a combination of 2'-FL and DFL.

5. The non-therapeutic use according to claim 1 or 2, wherein the fucosylated HMO is a combination of 2'-FL and LNFP-I.

6. The non-therapeutic use according to any of the preceding claims, wherein the fucosylated HMO is prepared as a synthetic composition.

7. The non-therapeutic use according to claim 6 , wherein the fucosylated HMO is 2'-FL and is combined with LNT and/or LNnT.

8. The non-therapeutic use according to claim 6 or 7, wherein the fucosylated HMO is a combination of 2'-FL, DFL further combined with LNT and/or LNnT.

9. The non-therapeutic use of a fucosylated human milk oligosaccharides (HMOs) or a synthetic composition comprising or consisting essentially of one or more fucosylated human milk oligosaccharides according to any one of claims 1 to 8, in the dietary management of a non-infant human having fragmented sleep composed of frequent short bout of sleeps and absence of circadian cycle.

10. The non-therapeutic use of the fucosylated human milk oligosaccharide or the synthetic composition comprising or consisting essentially of one or more fucosylated human milk oligosaccharides according to any one of claims 1 to 9, wherein a daily dose of the fucosylated human milk oligosaccharide or synthetic composition contains 0.1 g to 10 g, more preferably 0.2 g to 7.5 g, for example 1 g to 5 g, of HMO.

11. The non-therapeutic use according to any of the preceding claims, wherein the non-infant human is administered the fucosylated human milk oligosaccharide for a period of at least 1 week, more preferably for at least 2 weeks.

12. The non-therapeutic use according to claims 10 or 11, wherein the non-infant human is administered a higher dose initially followed by a lower dose.

13. The non-therapeutic use according to any of the preceding claims, wherein the non-infant human is a child, a teenager, an adult or an elderly person.

## Patentansprüche

1. Nicht-therapeutische Verwendung eines fucosylierten humanen Milch-Oligosaccharids (HMO), vorzugsweise eines fucosylierten neutralen HMO, zur Unterstützung der Reifung oder Verbesserung von Schlafmustern bei einem menschlichen Nicht-Säugling, wobei die Reifung oder Verbesserung von Schlafmustern die Entwicklung eines Nacht- und Tagesschlafmusters von einem stärker fragmentierten Schlaf, der aus häufigem Kurzschlaf und Abwesenheit eines zirkadianen Zyklus besteht, hin zu einem Muster mit langem ununterbrochenen Nachtschlaf, begrenztem Tagesschlaf und Anwesenheit eines zirkadianen Zyklus beinhaltet.

2. Nicht-therapeutische Verwendung nach Anspruch 1, wobei das fucosylierte HMO 2'-FL, 3-FL, DFL, LNFP-I, LNFP-II, LNFP-III, LNFP-V, LNFP-VI oder eine beliebige Kombination davon ist.

3. Nicht-therapeutische Verwendung nach Anspruch 1 oder 2, wobei das fucosylierte HMO 2'-FL ist.

4. Nicht-therapeutische Verwendung nach Anspruch 1 oder 2, wobei das fucosylierte HMO eine Kombination von 2'-FL und DFL ist.

5. Nicht-therapeutische Verwendung nach Anspruch 1 oder 2, wobei das fucosylierte HMO eine Kombination von 2'-FL und LNFP-I ist.

6. Nicht-therapeutische Verwendung nach einem der vorhergehenden Ansprüche, wobei das fucosylierte HMO als eine synthetische Zusammensetzung hergestellt wird.

7. Nicht-therapeutische Verwendung nach Anspruch 6, wobei das fucosylierte HMO 2'-FL ist und mit LNT und/oder LNnT kombiniert wird.

8. Nicht-therapeutische Verwendung nach Anspruch 6 oder 7, wobei das fucosylierte HMO eine Kombination von 2'-FL, DFL, ferner kombiniert mit LNT und/oder LNnT, ist.

9. Nicht-therapeutische Verwendung eines fucosylierten humanen Milch-Oligosaccharids (HMO) oder einer synthetischen Zusammensetzung, die ein oder mehrere fucosylierte humane Milch-Oligosaccharide umfasst oder im Wesentlichen daraus besteht, nach einem der Ansprüche 1 bis 8 bei der diätetischen Behandlung eines menschlichen Nicht-Säuglings mit fragmentiertem Schlaf, der aus häufigem Kurzschlaf und Abwesenheit eines zirkadianen Zyklus besteht.

10. Nicht-therapeutische Verwendung des fucosylierten humanen Milch-Oligosaccharids oder der synthetischen Zusammensetzung, die ein oder mehrere fucosylierte humane Milch-Oligosaccharide umfasst oder im Wesentlichen daraus besteht, nach einem der Ansprüche 1 bis 9, wobei eine Tagesdosis des fucosylierten humanen Milch-Oligosaccharids oder der synthetischen Zusammensetzung 0,1 g bis 10 g, stärker bevorzugt 0,2 g bis 7,5 g, zum Beispiel 1 g bis 5 g, HMO enthält.

11. Nicht-therapeutische Verwendung nach einem der vorhergehenden Ansprüche, wobei dem menschlichen Nicht-Säugling das fucosylierte humane Milch-Oligosaccharid für einen Zeitraum von mindestens 1 Woche, stärker bevorzugt für mindestens 2 Wochen verabreicht wird.

12. Nicht-therapeutische Verwendung nach Anspruch 10 oder 11, wobei dem menschlichen Nicht-Säugling anfangs eine höhere Dosis verabreicht wird, gefolgt von einer niedrigeren Dosis.

13. Nicht-therapeutische Verwendung nach einem der vorhergehenden Ansprüche, wobei es sich bei dem menschlichen Nicht-Säugling um ein Kind, einen Teenager, einen Erwachsenen oder eine ältere Person handelt.

## Revendications

1. Utilisation non thérapeutique d'un oligosaccharide de lait humain (HMO) fucosylé, de préférence d'un HMO neutre fucosylé, pour soutenir la maturation ou l'amélioration de rythmes de sommeil, chez un être humain qui n'est pas un nourrisson, dans laquelle la maturation ou l'amélioration de rythmes de sommeil implique le développement d'un rythme de sommeil nocturne et diurne pour aller d'un sommeil plus fragmenté composé de fréquents épisodes courts de sommeil et d'une absence de cycle circadien vers un rythme comportant un long sommeil nocturne ininterrompu, un sommeil diurne limité et la présence d'un cycle circadien.

2. Utilisation non thérapeutique selon la revendication 1, dans laquelle le HMO fucosylé est le 2'-FL, le 3-FL, le DFL, le LNFP-I, le LNFP-II, le LNFP-III, le LNFP-V, le LNFP-VI ou une quelconque combinaison de ceux-ci.

3. Utilisation non thérapeutique selon la revendication 1 ou 2, dans laquelle le HMO fucosylé est le 2'-FL.

4. Utilisation non thérapeutique selon la revendication 1 ou 2, dans laquelle le HMO fucosylé est une combinaison de 2'-FL et de DFL.

5. Utilisation non thérapeutique selon la revendication 1 ou 2, dans laquelle le HMO fucosylé est une combinaison de 2'-FL et de LNFP-I.

6. Utilisation non thérapeutique selon l'une quelconque des revendications précédentes, dans laquelle le HMO fucosylé est préparé sous forme d'une composition synthétique.

7. Utilisation non thérapeutique selon la revendication 6, dans laquelle le HMO fucosylé est le 2'-FL et est combiné avec du LNT et/ou du LNnT.

8. Utilisation non thérapeutique selon la revendication 6 ou 7, dans laquelle le HMO fucosylé est une combinaison de 2'-FL et de DFL en outre combinée avec du LNT et/ou du LNnT.

9. Utilisation non thérapeutique d'un oligosaccharide de lait humain (HMO) fucosylés ou d'une composition synthétique comprenant un ou plusieurs oligosaccharides de lait humain fucosylés ou constituée essentiellement de ceux-ci selon l'une quelconque des revendications 1 à 8, dans la prise en charge diététique d'un être humain qui n'est pas un nourrisson ayant un sommeil fragmenté composé de fréquents épisodes courts de sommeil et d'une absence de cycle circadien.

10. Utilisation non thérapeutique de l'oligosaccharide de lait humain fucosylé ou de la composition synthétique comprenant un ou plusieurs oligosaccharides de lait humain fucosylés ou constituée essentiellement de ceux-ci selon l'une quelconque des revendications 1 à 9, dans laquelle une dose quotidienne de l'oligosaccharide de lait humain fucosylé ou de la composition synthétique contient 0,1 g à 10 g, plus préférablement 0,2 g à 7,5 g, par exemple 1 g à 5 g, de HMO.

11. Utilisation non thérapeutique selon l'une quelconque des revendications précédentes, dans laquelle on administre l'oligosaccharide de lait humain fucosylé à l'être humain qui n'est pas un nourrisson sur une durée d'au moins 1 semaine, plus préférablement pendant au moins 2 semaines.

12. Utilisation non thérapeutique selon les revendications 10 ou 11, dans laquelle on administre initialement à l'être humain qui n'est pas un nourrisson une dose plus élevée suivie d'une dose plus faible.

13. Utilisation non thérapeutique selon l'une quelconque des revendications précédentes, dans laquelle l'être humain qui n'est pas un nourrisson est un enfant, un adolescent, un adulte ou une personne âgée.
